(19) Europäisches Patentamt
European Patent Office
Office européen des brevets

(11) **EP 2 081 428 B1**

(12) **EUROPEAN PATENT SPECIFICATION**

(45) Date of publication and mention
of the grant of the patent:
**15.05.2013 Bulletin 2013/20**

(51) Int Cl.:
***A01N 37/12*** (2006.01)

(21) Application number: **07804980.6**

(86) International application number:
**PCT/IB2007/002802**

(22) Date of filing: **19.09.2007**

(87) International publication number:
**WO 2008/038108 (03.04.2008 Gazette 2008/14)**

(54) **ANTIPEDICULOSIS COMPOSITION HAVING A LICE-SUFFOCATING ACTIVITY**

ZUSAMMENSETZUNG GEGEN PEDIKULOSE MIT LÄUSE-ERSTICKENDER WIRKUNG

COMPOSITION ANTI-PÉDICULOSE AGISSANT PAR SUFFOCATION DES POUX

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.09.2006 IT MI20061825**

(43) Date of publication of application:
**29.07.2009 Bulletin 2009/31**

(73) Proprietor: **Giuliani S.p.A.**
**20129 Milano (IT)**

(72) Inventors:
• **GIULIANI, Giammaria**
**I-20123 Milano (IT)**
• **BENEDUSI, Anna**
**I-20124 Milano (IT)**

(74) Representative: **Finetti, Claudia et al**
**Barzanò & Zanardo Milano S.p.A.**
**Via Borgonuovo 10**
**20121 Milano (IT)**

(56) References cited:
EP-A- 1 308 153        EP-A- 1 310 239
EP-A- 1 609 362        WO-A-00/72814
WO-A-01/60163          WO-A-2005/027636
WO-A-2006/000335       US-A1- 2002 143 203
US-A1- 2004 161 441

• **"MIGLYOL 8810" 5 January 2002 (2002-01-05), SASOL NORTH AMERICA , XP002514459 the whole document**

• **DATABASE BIOSIS [Online] BIOSCIENCES INFORMATION SERVICE, PHILADELPHIA, PA, US; December 2000 (2000-12), MORSY TOSSON A ET AL: "Evaluation of the in-vitro pediculicidal action of four known insecticides and three medicinal plant extracts" XP002514460 Database accession no. PREV200100024598 & JOURNAL OF THE EGYPTIAN SOCIETY OF PARASITOLOGY, vol. 30, no. 3, December 2000 (2000-12), pages 699-708, ISSN: 1110-0583**
• **DATABASE MEDLINE [Online] US NATIONAL LIBRARY OF MEDICINE (NLM), BETHESDA, MD, US; December 2005 (2005-12), YANG YOUNG-CHEOL ET AL: "Ovicidal and adulticidal activities of Cinnamomum zeylanicum bark essential oil compounds and related compounds against Pediculus humanus capitis (Anoplura: Pediculicidae)." XP002514461 Database accession no. NLM16188263 & INTERNATIONAL JOURNAL FOR PARASITOLOGY DEC 2005, vol. 35, no. 14, December 2005 (2005-12), pages 1595-1600, ISSN: 0020-7519**
• **DATABASE BIOSIS [Online] BIOSCIENCES INFORMATION SERVICE, PHILADELPHIA, PA, US; December 2005 (2005-12), YANG YOUNG-CHEOL ET AL: "Ovicidal and adulticidal activities of Cinnamomum zeylanicum bark essential oil compounds and related compounds against Pediculus humanus capitis (Anoplura : Pediculicidae)" XP002514462 Database accession no. PREV200600166601 & INTERNATIONAL JOURNAL FOR PARASITOLOGY, vol. 35, no. 14, December 2005 (2005-12), pages 1595-1600, ISSN: 0020-7519**

- **DATABASE BIOSIS [Online] BIOSCIENCES INFORMATION SERVICE, PHILADELPHIA, PA, US; March 2004 (2004-03), MILLS CLIVE ET AL: "Inhibition of acetylcholinesterase by Tea Tree oil" XP002514463 Database accession no. PREV200400311022 & JOURNAL OF PHARMACY AND PHARMACOLOGY, vol. 56, no. 3, March 2004 (2004-03), pages 375-379, ISSN: 0022-3573**

Remarks:

The file contains technical information submitted after the application was filed and not included in this specification

**Description**

The present invention relates to an antipediculosis composition having a lice-suffocating activity.

The present invention derives from the field of cosmetic products and medical-surgical aids/medical expedients having an antipediculosis action.

In particular, the present invention relates to preparations for external use having a pediculicide, ovocide or preventive action against parasitic infestations of the scalp.

The term pediculosis refers to lice (Pediculus Humanus) infestations, known parasites which nest and develop only at a scalp level.

Lice have dimensions of about 2-3 mm, and have a flat yellow-brownish body, with six arms at whose ends there is a hooked nail used for attaching themselves to the hair. The bucal apparatus consists of a stinging rostrum suitable for suction. Once the rostrum has penetrated the skin it fixes itself with small moveable teeth.

While biting, lice secrete a substance which has an anaesthetizing effect on the skin so as not to irritate the hosting organism and consequently limit any adverse reaction.

Lice also inject an anticoagulating substance which fluidifies the blood during suction allow a prolonged suction for several hours. The quantity of blood sucked is about 1 mg, extremely high with respect to the parasitic mass. Lice feed twice a day for the whole of their lives. Their head has two small feelers whose function is to detect the temperature and direct the parasite towards finding: food and a partner.

Lice also have a rudimental vision system whose purpose is probably only that of perceiving light variations, as the eggs are deposited in the dark.

The reproduction cycle of lice derives from the nits (eggs) which are typically found in the retroauricular region and on the nape to which they attached thanks to an adhesive substance which they secrete.

The eggs hatch after a week and the louse begins to develop thanks to the supply of blood which it sucks by biting the scalp and causing strong itching.

The eggs, resistant to many insecticides, are attached to the hair root by means of a viscous secretion insoluble in water. The eggs hatch after about ten days, the small louse becomes reproductive after two weeks during which it moults three times. The life duration of lice is twenty days for males and forty days for females.

The correct temperature of the hosting organism is an extremely important factor, as lice immediately abandon the head when there is a strong thermal rise. The efficacy of a louse's grip on hair is a very important factor for its survival due to the fact that outside of its habitat it would only have a few hours of life as it would die of hunger and cold.

In order to ensure survival of the species, the parasite has had to develop its reproduction capacity. By laying about ten eggs a day for the whole of its existence, the female can in fact ensure over three hundred direct descendents. In a month, each female can reproduce in an overall descent of about 45,000 individuals, which after two months would reach the frightening quantity of 6,750,000. Fortunately in reality not all the individuals survive, others are eliminated with brushing or scratching. Lice represent one of the few living species which have not undergone changes with variations in the climate and daily customs of human beings.

The scalp is in fact a small ecological niche which has only undergone minimum changes over the centuries; the increased number of human beings has represented, for this parasite, a significantly greater opportunity for life and reproduction.

There are many reasons for the recurrence of this troublesome infestation : their efficient reproduction capacity; dispersion in the environment due to brushing, scratching, diving; the promiscuous use of handkerchiefs, armchairs, headrests, hats, caps, combs ...; the capacity, if expelled, of finding a new hosting organism.

The probability of contagion in view of present-day life customs have increased enormously with respect to the past; long permanence in crowded places such as working environments, schools, means of public transport and gymnasiums were not in fact part of the daily customs of our ancestors. Children have a greater probability of contagion due to their games and intenser social life.

Old remedies are known for the treatment of lice, such as washing the hair with vinegar to detach the eggs and shaving the scalp. The use of mercury ointments and DDT are also known.

Various specific biocide products are currently present on the market for the treatment of lice infestations, based on highly toxic active principles such as malathion, d-phenotrin, pyrethrin and piperonyl butoxide.

The use of these products however is not without drawbacks, among which a certain degree of toxicity and the risk of triggering allergy reactions which in many cases cause interruption and consequently unsuccessful disinfesting treatment.

Furthermore, no treatment is effective from the first application and this must consequently be repeated at a distance of a week to eliminate the larvae born from the surviving eggs.

WO0072814 discloses compositions for the removal of ecto-parasites comprising 6-18% emollient agents, including inter alia dicaprylate/dicaprate esters.

WO2005/027636 discloses compositions comprising fatty acid esters of mono-, di-, tri-, or poly-hydric alcohols for control of ecto-parasites. EP1308153 discloses insect-repellent sun-cream, comprising butyleneglycol dicaprylate/dicaprate as oil component.

In Italy, moreover, as also in the United States, England and other countries, it has been found that there is a growing resistance on the part of lice to the active principles so far used, with a consequent ineffective treatment of about 1 child out of 4.

The necessity is therefore felt in the present state of the art to avail of new products with an antipediculosis action.

One of the objectives of the present invention consequently consists in providing an antipediculosis composition which is highly effective in treating lice infestations, and which has a low toxicity towards the human organism.

A further objective of the present invention consists in providing an antipediculosis composition which is capable of also efficaciously eliminating forms of lice and larvae which are resistant to the biocide products currently present on the market.

A further objective of the present invention consists in providing a preparation for disinfesting the scalp from lice and larvae whose use involves low risks of developing allergy-type reactions which can cause interruption of the treatment.

The invention relates to the use of butylene glycol dicaprate-dicaprylate for the production of a composition for the external treatment of infestations on the part of ectoparasites, wherein said butylene glycol dicaprate-dicaprylate is present in a quantity ≥ 20% by weight, advantageously ≥ 40% by weight in said composition.

The applicant has now found that butylene glycol dicaprate-dicaprylate, an oily compound which has so far been used as an oily component in formulations for cosmetic use, unexpectedly exerts an antipediculosis action. The term antipediculosis means designating and comprising both a direct disinfesting action on parasites, typically lice, and also a direct ovocide action on the larvae and eggs.

The applicant has also found that this pediculicide/ovocide action can mainly be attributed to a suffocating action and inhibition of the flow of oxygen due to the volatility in gaseous phase and the filmogen effect of butylene glycol dicaprate-dicaprylate on ectoparasites in general and in particular on the most common types of lice which infest the human population.

It has also been found that the external use of the active principle butylene glycol dicaprate-dicaprylate implies low risks of toxicity and a reduced incidence of possible side-effects particularly of the allergy type.

According to an embodiment of the present invention, the use is envisaged of butylene glycol dicaprate-dicaprylate in the cosmetic treatment of the scalp infested by ectoparasites, in particular lice and/or eggs/larvae.

According to this embodiment of the invention, the active principle butylene glycol dicaprate-dicaprylate is formulated in a preparation containing one or more cosmetically acceptable carriers or excipients.

According to an embodiment, this cosmetic composition comprises urea, lactic acid and arginine. The kera-toplastic action of the urea preferably present in a high concentration, stabilized by the lactic acid-arginine buffer, allows a softening effect of the hair and facilitated detachment of any material adhering to it.

According to another embodiment, the presence of a penetration enhancer is envisaged, such as, for example, pentylene glycol, ethylhexyl glycerin, emulsifying agents with an oleylic chain, preferably having an antibacterial activity, which allows both diffusion in depth and the solubilization of possible further active principles of a vegetable origin, such as for example, anethol, terpineol, Neem oil, cinnamon oil, Andiroba oil, melaleuca oil.

Another aspect of the invention envisages the use of butylene glycol dicaprate-dicaprylate for the production of a medical-surgical aid for the treatment of infestations on the part of ectoparasites and lice in particular.

The form of biphasic extemporary emulsion allows the content in active principles to be optimized in the two separate phases and maintains the extreme distributing capacity of the product on the skin.

According to another aspect of the present invention, a composition is provided for disinfesting the scalp from lice and larvae characterized in that it comprises butylene glycol dicaprate-dicaprylate in an effective quantity from an antiparasitic point of view and a physiologically acceptable carrier wherein said butylene glycol dicaprate-dicaprylate is present in a quantity equal to or higher than 20% by weight and even more preferably equal to or higher than 40% by weight with respect to the total weight of the disinfesting composition.

The compositions for topic application of the invention can be in liquid form typically as water-oil emulsions, or as solutions in which the oily phase is highly dispersed in the aqueous phase or also as a shampoo. Semi-solid forms are also possible, such as gels, or also with a greater consistency such as creams, ointments, pomades and masks.

The compositions for local application of the invention can conveniently comprise additives commonly used in cosmetic or pharmaceutical preparations for local use, such as preservatives, stabilizers, emulsifying agents, buffers, dyes and other excipients commonly used in cosmetic/pharmaceutical preparation techniques.

In the case of liquid formulations, the synergic active principles of the invention can be conveniently dissolved in a cosmetically/pharmaceutically acceptable liquid carrier such as water, alcohol, hydro-alcohol, glycerin solution and other types suitable for local application.

The compositions of the invention in liquid form can be prepared, for example, by emulsifying the oil components such as butylene glycol dicaprate-dicaprylate in water and adding the suitable excipients.

The resulting mixture/emulsion can then be typically buffered to reach a pH level conveniently selected from 5 to 7 so as to be compatible with the pH of the skin and subsequently filtered and packaged in suitable containers ready for use.

According to a preferred embodiment, the composition of the invention is supplied as a biphasic water/oil lotion suitable for spray application resorting for example to a directional cannula. This form of administration is particularly suitable as it facilitates the physical suffocation of the lice.

The laying time of the emulsion on the scalp typically varies from 5-20 minutes and preferably is equal to about 15 minutes. After use, the oily phase can be easily removed by washing with a normal shampoo.

The composition for local use of the invention is used for applications, in an effective antiparasitic quantity, directly on the region of the scalp subjected to infestation or more conveniently on the whole of the head.

The composition of the invention can also be formulated as a detergent agent or shampoo whose composition includes surface-active agents, conditioning agents suitable for facilitating the elimination of lice, in addition to lenitive agents suitable for mitigating the irritation of the scalp and itching sensation.

The presence of mixtures of surface-active agents is suitable for facilitating the elimination of the antilice treating oil of the invention.

According to an embodiment, the formulation in shampoo form also contains an oil of a natural origin (Minga Andiroba) with a documented repellent activity with respect to lice.

According to another embodiment, the presence of apple vinegar is envisaged, which, by lowering the pH, creates a unfavourable nesting environment for lice.

According to this embodiment of the invention, the shampoo is also applied for preventing lice infestations during periods of the year in which the problem of infestations is more widely diffused.

If a formulation based on a solid or semi-solid is used, such as creams, hair balms, the butylene glycol dicaprate-dicaprylate is found in dispersed form in cosmetically/pharmaceutically acceptable carriers, commonly used for topic application.

According to another aspect of the invention, a cosmetic treatment method is provided, which comprises the local application, at the level of the scalp, of an effective quantity of an antipediculosis composition as described above.

According to another embodiment, a method is provided for disinfesting the scalp from parasites, in particular lice and larvae comprising the local application of an antipediculosis composition of the type previously described to a subject in need of treatment.

The following examples are provided for purely illustrative purposes of the present invention and should not be considered as limiting the protection scope as indicated in the above claims.

EXAMPLE 1

A composition of the invention is described, in the form of a biphasic water/oil lotion suitable for application on the scalp. Before use, it is advisable to shake the formulation in order to optimize the dispersion in water of the oily component.

Antipediculosis lotion

| Components (INCI denom.) | Function | Eff. % |
|---|---|---|
| Butylene glycol dicaprate-dicaprylate | Suffocating compound for lice | 48.30 |
| Anethol | Aromatizer | 12.00 |
| Ethylhexyl glycerin | Emollient | 9.99 |
| Pentylene glycol | Preservative | 5.40 |
| Urea | Active keratolytic | 5.00 |
| Sorbitane oleate | Carrier | 5.00 |
| Water | Carrier | 4.78 |
| Polysorbate 80 | Solubilizer | 4.01 |
| PPG-15 stearyl ether | Emollient | 1.35 |
| 4-terpineol | Active Antioxidant | 0.99 |
| Melia azadirachta seed oil | Active normalizer | 0.99 |
| Cinnamomum zeylanicum | Aromatizer | 0.99 |

(continued)

| Components (INCI denom.) | Function | Eff. % |
|---|---|---|
| Carapa guianensis seed oil | Emollient | 0.99 |
| CI 61565 | Liposoluble dye | 0.00 |
| Arginine | pH adjuster | 0.10 |
| Lactic acid | pH adjuster | 0.12 |
| CI 19140 | Water soluble dye | 0.00 |
| | | **100.00** |

Example 2

Antipediculosis lotion

| Components (INCI denom.) | Function | Eff. % |
|---|---|---|
| Water | Solvent | 42.92 |
| Acrylates C10/30 alkyl acrylates cross-polymer | Emulsifier | 0.35 |
| Butylene glycol dicaprate/di-caprylate | Suffocating compound for lice | 50.00 |
| Sodium hydroxide (18% solution) | pH adjuster | 0.35 |
| Urea | Active keratolytic | 5.00 |
| Glyceryl stearate | Emulsifier | 0.10 |
| Ceteth 20 | Emulsifier | 0.10 |
| Phenoxyethanol | Preservative | 0.80 |
| Methylparaben | Preservative | 0.20 |
| Ethylparaben | Preservative | 0.10 |
| Lactic acid | pH adjuster | 0.08 |
| | | **100.00** |

Example 3 (Comparative example, not forming part of the invention)

Antipediculosis oil shampoo

| Components (INCI denom.) | Function | Eff. % |
|---|---|---|
| Water | | 39.00 |
| PEG-7 glyceryl cocoate | Non-ionic surfactant | 20.00 |
| PEG-6 Caprylic capric glycerides | Non-ionic surfactant | 15.83 |
| Sodium Cocoamphoacetate | Amphoteric surfactant | 7.00 |
| Silicone quaternium-15 | Conditioning | 1.00 |
| Butylene glycol dicaprate/di-caprylate | Suffocating compound for lice | 10.00 |
| Anethol | Aromatizer | 4.00 |
| 4-terpineol | Active antioxidant | 0.99 |
| Melia azadirachta seed oil | Active normalizer | 0.99 |

(continued)

| Components (INCI denom.) | Function | Eff. % |
|---|---|---|
| Carapa quianensis seed oil | Emollient | 0.99 |
| Triethanolamine | pH adjuster | 0.10 |
| Lactic acid | pH adjuster | 0.10 |
| | | **100.00** |

Example 4

Concentrated antipediculosis ionic shampoo

| Components (INCI denom.) | Function | Eff. % |
|---|---|---|
| Butylene glycol dicaprate/dicaprylate | Suffocating compound for lice | 35.00 |
| Anethol | Aromatizer | 10.00 |
| Sodium laureth sulphate | Surfactant | 35.00 |
| Sodium lauroyl sarcosinate | Surfactant | 5.04 |
| Cocamydopropyl betaine | Surfactant | 10.00 |
| PEG-7 glyceryl cocoate | degreaser | 1.00 |
| 4-Terpineol | Active antioxidant | 0.99 |
| Melia azadirachta seed oil | Active normalizer | 0.99 |
| Cinnamomum zeylanicum | Aromatizer | 0.99 |
| Carapa quianensis seed oil | Emollient | 0.99 |
| | | **100.00** |

Example 5

Antipediculosis mousse

| Components (INCI denom.) | Function | Eff. % |
|---|---|---|
| Steareth-21 | Emulsifier | 5.00 |
| Steareth-2 | Emulsifier | 4.00 |
| PPG-15 STEARYL ETHER | | 4.00 |
| Butylene glycol dicaprate/dicaprylate | Suffocating compound for lice | 33.64 |
| Phenoxyethanol | Preservative | 1.00 |
| Imidazolydynyl Urea | Preservative | 0.05 |
| water | Carrier | 40.00 |
| 4-terpineol | Active antioxidant | 0.10 |
| Cinnamomum zeylanicum | Aromatizer | 0.99 |
| Sodium hydroxide (0.18 solution) | pH adjuster | 0.10 |
| Lactic acid | pH adjuster | 0.12 |
| Butane | Propellant | 6.00 |

7

(continued)

| Components (INCI denom.) | Function | Eff. % |
|---|---|---|
| Propane | Propellant | 1.00 |
| Isobutane | Propellant | 4.00 |
| | | **100.00** |

EXAMPLE 6

A composition in the form of a lotion according to example 1 was tested to evaluate the adulticide and ovicide activity in vitro on a pool of lice belonging to Pediculus humanus capitis. For this purpose, adults and eggs (nits) belonging to the test micro-organism were removed from the natural host (school children) and were treated with the substance under examination as such to simulate the conditions of use of the product. As a comparative material, two analogous leading products on the market were used, of which one based on natural substances (Paranix®) and one based on permethrins (MiLice®, positive control). The product tested proved to be effective in efficaciously eliminating (mortality > 90%) the adults treated already an hour after exposure of the test organism to the lotion. The product tested proved to be also effective in efficaciously eliminating (mortality >65%) the eggs treated 12 days after exposure of the test parasite to the lotion under examination. In view of the results of the efficacy test in vitro on: Pediculus humanus capitis, it is possible to classify the composition tested as a pediculicide with respect to both adults (adulticide) and eggs (ovicide). Pediculus humanus capitis, an obligate parasite microorganism which, in order to survive, feeds on human blood and prefers environmental conditions of temperature and humidity which are found at the level of the scalp, was used in the test.

Various studies currently effected in the whole world demonstrate how some strains of this parasite are developing a considerable resistance to biocides and how it is therefore necessary to test not only new formulations, but also the products already on the market to evaluate their efficacy.

In order to effect the experimentation in vitro, Pediculus humanus capitis taken from persons infested and not previously treated with other pediculicide products, was used as test system. The choice of the louse which infests the person's scalp was made considering the fact that a product specific for this type of insect was being tested.

The separate evaluation of the ovicide effectiveness is linked to the fact that the eggs of Pediculus humanus capitis are generally more difficult to eliminate and normally require a second treatment after a few days to be completely eliminated; this also explains why in the test, the efficacy criteria for the ovicide activity are less severe with respect to those of the adulticide activity, for which an effectiveness > 90% is required. The test was carried out under WHO conditions of use.

BIBLIOGRAPHIC REFERENCES

1. Standard Test Method for Determining the Effectiveness of Liquid, Gel, Cream, or Shampoo insecticides Against Adult Human Lice. Designation ASTM: E938 - 05
2. Standard Test Method for Determining the Effectiveness of Liquid, Gel, Cream, or Shampoo insecticides Against Adult Louse Ova. Designation ASTM: EI 517-99
3. WHO Model Prescribing Information. Drugs Used in Parasitic Diseases, 2nd Ed., World Health Organization, Geneva
4. Abbott, W.S., "A Method of Computing the Effectiveness of An Insecticide", 1925
5. Box, G., Hunter, 5., "Statistics for Experimenters", Wiley-1978.

ADULTICIDE EFFECTIVENESS TEST

TEST SYSTEM

The following micro-organisms were used for the experimentation: Pediculus humanus capitis. The lice were taken from school children (4-11 years old), after the reporting of infestation (pediculosis) to the National Health System. The lice were removed by an expert doctor and taken immediately to the laboratory for tests. The organisms were removed from dry hair using thick-toothed combs, they were placed in a plastic container JX10 cm (on which the date and hour of removal were recorded) and immediately transported to the test centre.

EQUIPMENT AND MATERIALS

- Tap water
- Distilled water
- Thermostat-regulated incubator (31°±1°C and 70% ± 10% R.H.)
- Chronometer
- Tweezers
- Test container (transparent plastic multidrop tray and transparent plastic stick with a circular shield)
- Cylindrical wooden applicator to which the infested tufts were attached
- Adhesive tape
- Thermostat-regulated bath (31° ± 1°C)
- Beaker (100-1,000 ml)
- Petri capsules (8.9 cm in diameter and 1.3 cm in depth)
- Absorbing towels
- Plastic containers (chemically inert 7x10 cm)
- Piece of dark fabric (4x4 cm)
- Heating plate
- Camelhair brush
- Thick-toothed comb

Structure of the study

The study was effected using distilled water as negative control. The substance was tested in replicas of 3, each replica containing 10 insects.

Negative control

Distilled water

PREPARATION OF THE TEST SAMPLE

10 ml of the substance being tested were poured into a 100 ml beaker and placed in a bath thermostat-regulated at 31° ± 1°C.
A 1000 ml beaker containing distilled water (washing solution) was kept in a bath thermostat-regulated at 31° ± 1°C.

METHOD EMBODIMENT

Exposure

3 replicas of 10 lice each were inserted on the bottom of the test container and the organisms were kept anchored to the bottom by means of a suitable plastic stick.
The test container was inserted in the 100 ml beaker at 31° ± 1°C containing the pediculicide substance. The organisms were left in contact with the substance for a period of 15 minutes.

Washing

The test container was removed from the beaker and the remaining liquid was eliminated from the test container.
The test container was inserted in the 1,000 ml beaker containing distilled water at 31° ± 1°C and stirred. It was removed after 1 minute and the lice were delicately rinsed under a jet of distilled water (31° ± 1°C) for 1 minute. The excess water was removed.

Incubation

Each replica was transferred onto a piece of dark fabric 4x4 and inserted in a Petri capsule. The Petri capsules with the treated lice were placed in an incubator thermostat-regulated at 31° ± 1°C and 70% ± 5% R.H.

For the controls, the procedure was repeated as such except for the fact that the product being tested was substituted by distilled water (negative control).

The mortality of the lice was evaluated 15 minutes, 30 minutes, 1 hour, 3 hours, 6 hours and 24 hours after the end of the treatment.

For this purpose, the lice situated on the fabric were exposed to the heat of a plate heated to 37° ± 1°C. The lice already dead or dying were not able to move towards the heat source within 5 minutes of exposure to heat.

CALCULATION-EXPRESSION OF THE RESULTS

For each result, the average cumulative mortality percentage of the substance tested and negative control was evaluated, by adding the sum of the already dead or dying lice. This value was corrected using the Abbot formula.

```
Corrected mortality = [(% Survivors in the Negative control) - Survivors in the Test)] I (% Survivors in the

Negative Control) X100
```

TEST VALIDITY CRITERIA

For each contact time, the test can be considered valid if the average cumulative mortality for the negative control is less than 15% and if the average cumulative mortality for the positive control is higher than 90% starting from 1 hour after treatment.

ACCEPTABILITY AND EFFECTIVENESS CRITERIA

The substance under examination can be considered pediculicide if the corrected average mortality is equal to or higher than 90% within 6 hours of treatment.

RESULTS

The composition of example 1 showed an adulticide effectiveness equal to 93% after 1 hour from the end of the treatment and equal to 100% after 3 hours from the end of the treatment.

The following table indicates the data relating to the cumulative mortality percentage of the lice treated towards the comparative materials and negative control.

TABLE 1: average accumulative mortality percentage (%) - treatment with lotion versus negative control

|  | t0 | 15 min | 30 min | 1h | 3h | 6h | 24h |
|---|---|---|---|---|---|---|---|
| Biphasic antilice detergent | 0 | 53 | 80 | 93 | 100 | 100 | 100 |
| Paranix | 0 | 47 | 80 | 97 | 100 | 100 | 100 |
| Milice (Pc) | 0 | 60 | 85 | 100 | 100 | 100 | 100 |
| Distilled water (Nc) | 0 | 0 | 0 | 0 | 0 | 3.3 | 17 |
| Pc = positive control Nc = negative control | | | | | | | |

The corrected mortality (Abbott formula) of the lotion according to Example 1 proves to be equal to 93% after an hour of incubation and equal to 100% after 3 and 6 hours of incubation, thus satisfying the effectiveness criteria.

Furthermore the validity criteria of the test up to 6 h are fully satisfied.

DEVIATIONS

No deviation was observed during the study.

CONCLUSIONS

The effectiveness test in vitro on Pediculus humanus capitis allowed the composition in the form of a lotion according to Example 1 to be classified as an adulticide.

OVICIDE EFFECTIVENESS TEST

1. TEST SYSTEM

1.1 Target organisms

The following organisms are used for the experimentation:

Pediculus-humanus-capitis (eggs). The lice were taken from school children (4-11 years old), after the reporting of infestation (pediculosis) to the National Health System. The lice were removed by an expert doctor and taken immediately to the laboratory for tests. The organisms were removed together with the infested tufts of hair and were placed in a plastic container 7X10 cm (on which the date and hour of removal were recorded) and immediately transported to the test centre.

2. EQUIPMENT AND MATERIALS

- Tap water
- Distilled water
- Thermostat-regulated incubator
- Chronometer
- Tweezers
- Cylindrical wooden applicator to which the infested tufts were attached
- Adhesive tape
- Thermostat-regulated bath
- Beaker (100-1,000 ml)
- Petri capsules (8.9 cm in diameter and 1.3 cm in depth)
- Absorbing towels
- Plastic containers (chemically inert 7x10 cm)

EXPERIMENTAL DESIGN

Structure of the study

The study was effected using distilled water as negative control.
The substance was tested in replicas of 3, each replica containing 15 eggs.

Negative control

Distilled water

Positive control

Preparation of the test sample

60 ml of the substance being tested were poured into a 100 ml beaker and placed in a bath thermostat-regulated at $31° \pm 1°C$. A 1000 ml beaker containing distilled water (washing solution) was kept in a bath thermostat-regulated at $31° \pm 1°C$.

TEST EMBODIMENT

Exposure

Each replica of 10 eggs consisting of 1 to 3 tufts and containing 10 eggs was attached to a wooden stick-applicator by means of adhesive tape. The samples of the substance being tested were brought to 31° ± 1°C in thermostat-regulated bath. The applicator with the eggs was inserted in the 100 ml beaker at 32°C containing the ovicide substance. The organisms remained in contact with the substance for a period of 15 minutes (on the basis of the Client's indications) and the applicator was then removed from the beaker.

The tufts were washed in 900 ml of tap water at 31° ± 1°C and stirred by means of an "up-and-down" movement of the applicator to simulate live rinsing.

After 1 minute the eggs were delicately rinsed in the 1,000 ml beaker for a minute and the excess water was removed.

The tufts with the eggs attached were placed in a Petri capsule and incubated at 31.7° ± 0.5°C and 60% ± 10% R.H.

For the controls, the procedure was repeated as such except for the fact that the product being tested was substituted by distilled water (negative control).

OBSERVATIONS

When all the eggs of the negative control had opened (after about 12 days), the replicas were examined under a microscope (30X) to determine the number of eggs which had opened regularly and those which, on the contrary, had not. The latter were classified as dead organisms. The partially opened eggs (emerging) are classified as dead organisms.

CALCULATION AND EXPRESSION OF THE RESULTS

Evaluate the average percentage mortality of the substance tested and negative control, by adding the unopened or partially opened eggs and correcting the mortality values for the positive control and for the substance under examination using the Abbott formula. Corrected mortality = [(% Survivors in the Negative control) - Survivors in the Test)] J/ (% Survivors in the Negative Control) X 100

TEST VALIDITY CRITERIA

The test can be considered valid if, at the end of the treatment, the average cumulative mortality for the negative control is less than 15% and for the positive control it is higher than 65%.

ACCEPTABILITY AND EFFECTIVENESS CRITERIA

The substance under examination can be considered an ovicide if the average corrected mortality is equal to or higher than 65%.

RESULTS

The lotion of Example 1 showed an ovicide effectiveness > 65% 12 days after the end of the treatment. Furthermore, the substance being examined, under the experimental conditions adopted, proved to have a higher ovicide effectiveness with respect to that of the leading comparative materials on the market. Table 2 below indicates the data relating to the cumulative mortality percentage of the eggs treated versus the comparative materials and the negative control:

TABLE 2: average cumulative mortality percentage (%) corrected after treatment with lotion vs negative control and comparative material

| | |
|---|---|
| Biphasic antilice detergent | 86% |
| Paranix | 74.8% |
| Milice (pc) | 78.5% |

(continued)

| Distilled water (nc) | 7.0% |
|---|---|
| pc = positive control<br>nc = negative control | |

The corrected mortality (Abbott formula) of the antilice lotion of Example 1 proves to be higher than 65% after 12 days of incubation: the effectiveness criteria are therefore satisfactory. The test validity criteria also prove to be fully satisfactory, as at all the observation times, the negative control has an cumulative mortality percentage < 15% and the positive control has an cumulative mortality percentage higher than 65%.

DEVIATIONS

No deviation was observed during this study.

CONCLUSIONS

The effectiveness test in vitro on Pediculus humanus capitis nits allowed the lotion according to Example 1 to be classified as an OVICIDE.
The results obtained are summarized in the following table.

TABLE 3

| | Product tested | Nr. of nits tested | Number of nits open/dead after 12 days | | |
|---|---|---|---|---|---|
| | | | Vital nits | Non-vital nits | |
| | | | Open | Not open | Partially open |
| Biphasic detergent | replica 1 | 10 | 2 | 5 | 3 |
| | replica 2 | 10 | 2 | 7 | 1 |
| | replica 3 | 10 | 0 | 8 | 2 |
| | average | 10 | 1.3 | 6.7 | 2 |
| Paranix | replica 1 | 10 | 2 | 6 | 2 |
| | replica 2 | 10 | 3 | 7 | 0 |
| | replica 3 | 10 | 2 | 6 | 2 |
| | average | 10 | 2.3 | 6.3 | 1.3 |
| | | | | | |
| Milice | replica 1 | 10 | 2 | 6 | 2 |
| | replica 2 | 10 | 2 | 6 | 2 |
| | replica 3 | 10 | 2 | 6 | 2 |
| | average | 10 | 2 | 6 | 2 |

| | Product tested | Nr. of lice tested | Number of dead lice after: | | | | | | |
|---|---|---|---|---|---|---|---|---|---|
| | | | 15 min. | 30 min. | 1 hr | 3 hr | 6 hr | -- | 24 hr |
| Negative control | replica 1 | 10 | 0 | 0 | 0 | 0 | 1 | -- | 2 |
| | replica 2 | 10 | 0 | 0 | 0 | 0 | 0 | -- | 1 |
| | replica 3 | 10 | 0 | 0 | 0 | 0 | 0 | -- | 2 |
| | average | 10 | 0 | 0 | 0 | 0 | 0.3 | -- | 1.7 |

**Claims**

1. Use of butylene glycol dicaprate-dicaprylate for the production of a composition for the external treatment of infestations on the part of ectoparasites, wherein said butylene glycol dicaprate-dicaprylate is present in a quantity ≥ 20% by weight, advantageously ≥ 40% by weight in said composition.

2. Use according to claim 1, **characterized in that** said composition is a medical expedient or medical-surgical aid for human/veterinary use or a cosmetic composition for the treatment of lice and/or nits.

3. Use according to any of claims 1-2, wherein said composition is in liquid form.

4. Use according to claim 3, wherein said composition is in the form of a biphasic water/oil or oil/water lotion.

5. Use according to claim 3, wherein said composition is in the form of a shampoo or mousse.

6. Use according to any of claims 1-2, wherein said composition is in solid or semi-solid form.

7. Use according to claim 6, wherein said composition is in the form of a hair cream or paste.

8. Use according to any of claims 1-7, wherein said composition comprises a further antipediculosis active principle selected from the group of: anethol, terpineol, Neem oil, urea, Melaleuca oil, Cinnamon oil, Andiroba oil and mixtures thereof.

9. An antipediculosis composition, **characterized in that** it comprises butylene glycol dicaprate-dicaprylate in an effective quantity from an antiparasitic point of view and a physiologically acceptable carrier wherein said butylene glycol dicaprate-dicaprylate is present in a quantity ≥ 20% by weight, advantageously ≥ 40% by weight

10. The composition according to claim 9, **characterized in that** it is in liquid form selected from a biphasic water/oil or oil/water lotion, mousse, shampoo.

11. The composition according to any of claims 9-10, **characterized in that** it comprises at least a further antipediculosis active principle selected from Melaleuca oil, anethol, terpineol, Neem oil, Cinnamon oil/essence, urea, Andiroba oil and/or mixtures thereof.

12. The composition according to any of claims 9-11, **characterized in that** it also comprises a penetration enhancer selected from pentylene glycol, ethylhexyl glycerin, emulsifying agents with an oleylic chain and mixtures thereof.

13. The composition according to any of claims 9-12 in the form of a biphasic lotion having the following composition:

| | |
|---|---|
| Butylene glycol dicaprate-dicaprylate | 48.30% |
| Anethol | 12.00% |
| Ethylhexyl glycerin | 9.99% |
| Pentylene glycol | 5.40% |
| Urea | 5.00% |
| Sorbitane oleate | 5.00% |
| Water | 4.78% |
| Polysorbate 80 | 4.01% |
| PPG-15 stearyl ether | 1.35% |
| 4-terpineol | 0.99% |
| Melia azadirachta (Neem tree) seed oil | 0.99% |
| Cinnamomum zeylanicum | 0.99% |

(continued)

| | |
|---|---|
| Carapa guianensis (Andiroba) seed oil | 0.99 |
| Arginine | 0.10 |
| Lactic acid | 0.12 |
| | **100.00** |

## Patentansprüche

1. Verwendung von Butylenglycol Dicaprylat/Dicaprat zur Herstellung einer Zusammensetzung für die äußerliche Behandlung von Infestationen seitens Ektoparasiten, wobei dieses Butylenglycol Dicaprylat/Dicaprat in einer Menge ≥ 20 Gew.-%, vorteilhafterweise ≥ 40 Gew.-%, in dieser Zusammensetzung vorliegt.

2. Verwendung nach Anspruch 1, **dadurch gekennzeichnet, dass** die Zusammensetzung ein medizinisches Mittel oder ein medizinisch-chirurgisches Hilfsmittel für human- oder veterinärmedizinische Anwendungen oder eine kosmetische Zusammensetzung zur Behandlung von Läusen und/oder Nissen ist.

3. Verwendung nach einem der Ansprüche 1 bis 2, wobei die Zusammensetzung in flüssiger Form vorliegt.

4. Verwendung nach Anspruch 3, wobei die Zusammensetzung in Form einer zweiphasigen Wasser/Öl- oder Öl/Wasser-Lotion vorliegt.

5. Verwendung nach Anspruch 3, wobei die Zusammensetzung in Form eines Shampoos oder Schaums vorliegt.

6. Verwendung nach einem der Ansprüche 1 bis 2, wobei die Zusammensetzung in fester oder halbfester Form vorliegt.

7. Verwendung nach Anspruch 6, wobei die Zusammensetzung in Form einer Haarcreme oder Paste vorliegt.

8. Verwendung nach einem der Ansprüche 1 bis 7, wobei die Zusammensetzung ein weiteres Wirkungsprinzip gegen Pedikulose umfasst, das aus der Gruppe ausgewählt ist, die aus Folgendem besteht: Anethol, Terpineol, Niemöl, Harnstoff, Teebaumöl (Melaleuca), Zimtöl, Andirobaöl und Mischungen davon.

9. Zusammensetzung zur Behandlung von Pedikulose, **dadurch gekennzeichnet, dass** sie Butylenglycol Dicaprylat/Dicaprat in einer in antiparasitärer Hinsicht wirksamen Menge und einen physiologisch akzeptablen Träger umfasst, wobei das Butylenglycol Dicaprylat/Dicaprat in einer Menge ≥ 20Gew.-%, vorteilhafterweise ≥ 40 Gew.-%, vorliegt.

10. Zusammensetzung nach Anspruch 9, **dadurch gekennzeichnet, dass** sie in einer flüssigen Form vorliegt, die aus zweiphasiger Wasser/Öl- oder Öl/Wasser-Lotion, Schaum oder Shampoo ausgewählt ist.

11. Zusammensetzung nach einem der Ansprüche 9 bis 10, **dadurch gekennzeichnet, dass** sie mindestens ein weiteres Wirkungsprinzip gegen Pedikulose umfasst, das aus Teebaumöl (Melaleuca), Anethol, Terpineol, Niemöl, Zimtöl/-essenz, Harnstoff, Andirobaöl und/oder Mischungen davon ausgewählt ist.

12. Zusammensetzung nach einem der Ansprüche 9 bis 11, **dadurch gekennzeichnet, dass** sie außerdem einen Penetrationsverstärker umfasst, der aus Pentylenglycol, Ethylhexylglycerin, Emulgatoren mit einer Oleyl-Kette und Mischungen davon ausgewählt ist.

13. Zusammensetzung nach einem der Ansprüche 9 bis 12 in Form einer zweiphasigen Lotion mit der folgenden Zusammensetzung:

| | |
|---|---|
| Butylenglycol Dicaprylat/Dicaprat | 48,30% |
| Anethol | 12,00% |
| Ethylhexylglycerin | 9,99% |

(fortgesetzt)

| | |
|---|---|
| Pentylenglycol | 5,40% |
| Harnstoff | 5,00% |
| Sorbitanoleat | 5,00% |
| Wasser | 4,78% |
| Polysorbat 80 | 4,01% |
| PPG-15 Stearylether | 1,35% |
| 4-Terpineol | 0,99% |
| Niembaum- (Melia azadirachta) -Saatöl | 0,99% |
| Cinnamomum zeylanicum | 0,99% |
| Andiroba- (Carapa guianensis) -Saatöl | 0,99 |
| Arginin | 0,10 |
| Milchsäure | 0,12 |
| | 100,00 |

**Revendications**

1. Utilisation de dicaprate-dicaprylate de butylène glycol pour la production d'une composition pour le traitement externe d'infestations de la part d'ectoparasites, dans laquelle ledit dicaprate-dicaprylate de butylène glycol est présent dans une quantité ≥ 20% en poids, avantageusement ≥ 40% en poids dans ladite composition.

2. Utilisation selon la revendication 1, **caractérisée en ce que** ladite composition est une aide de secours médical ou médico-chirurgicale pour usage humain/vétérinaire ou une composition cosmétique pour le traitement de poux et/ou de lentes.

3. Utilisation selon l'une quelconque des revendications 1-2, dans laquelle ladite composition est sous forme liquide.

4. Utilisation selon la revendication 3, dans laquelle ladite composition est sous la forme d'une lotion eau/huile ou huile/eau bi-phasique.

5. Utilisation selon la revendication 3, dans laquelle ladite composition est sous la forme d'un shampoing ou d'une mousse.

6. Utilisation selon l'une quelconque des revendications 1-2, dans laquelle ladite composition est sous forme solide ou semi-solide.

7. Utilisation selon la revendication 6, dans laquelle ladite composition est sous la forme d'une crème ou pâte pour cheveux.

8. Utilisation selon l'une quelconque des revendications 1-7, dans laquelle ladite composition comprend un autre principe actif anti-pédiculose sélectionné dans le groupe comprenant : anéthol, terpinéol, huile de l'arbre de Neem, urée, huile de melaleuca, huile de cannelle, huile d'Andiroba et des mélanges de ceux-ci.

9. Composition anti-pédiculose, **caractérisée en ce qu'**elle comprend du dicaprate-dicaprylate de butylène glycol dans une quantité efficace d'un point de vue antiparasite et un support physiologiquement acceptable, dans laquelle ledit dicaprate-dicaprylate de butylène glycol est présent dans une quantité ≥ 20% en poids, avantageusement ≥ 40% en poids.

10. Composition selon la revendication 9, **caractérisée en ce qu'**elle est sous forme liquide sélectionnée parmi une lotion eau/huile ou huile/eau bi-phasique, une mousse, un shampoing.

**11.** Composition selon l'une quelconque des revendications 9-10, **caractérisée en ce qu'**elle comprend au moins un autre principe actif anti-pédiculose sélectionné parmi huile de melaleuca, anéthol, terpinéol, huile de l'arbre de Neem, huile/essence de cannelle, urée, huile d'Andiroba et/ou des mélanges de ceux-ci.

**12.** Composition selon l'une quelconque des revendications 9-11, **caractérisée en ce qu'**elle comprend également un promoteur de pénétration sélectionné parmi penthylène glycol, éthylhexylglycérine, agents émulsifiants avec chaîne oléylique et des mélanges de ceux-ci.

**13.** Composition selon l'une quelconque des revendications 9-12 sous la forme d'une lotion bi-phasique ayant la composition suivante :

| | |
|---|---|
| Dicaprate-dicaprylate de butylène glycol | 48,30% |
| Anethol | 12,00% |
| Ethylhexylglycérine | 9,99% |
| Penthylène glycol | 5,40% |
| Urée | 5,00% |
| Oléate de sorbitan | 5,00% |
| Eau | 4,78% |
| Polysorbate 80 | 4,01% |
| Ether stéarylique de PPG-15 | 1,35% |
| 4-terpinéol | 0,99% |
| Huile des graines de melia azadirachta (arbre de Neem) | 0,99% |
| Cinnamomum zeylanicum | 0,99% |
| Huile des graines de carapa guianensis (Andiroba) | 0,99 |
| Arginine | 0,10 |
| Acide lactique | 0,12 |
| | 100,00 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0072814 A
- WO 2005027636 A
- EP 1308153 A

**Non-patent literature cited in the description**

- Standard Test Method for Determining the Effectiveness of Liquid, Gel, Cream, or Shampoo insecticides Against Adult Louse Ova. *Designation ASTM: EI 517-99*
- WHO Model Prescribing Information. Drugs Used in Parasitic Diseases. World Health Organization
- **ABBOTT, W.S.** *A Method of Computing the Effectiveness of An Insecticide,* 1925